# EUROPEAN PATENT APPLICATION

(11) **EP 4 678 007 A1**
(43) Date of publication of application: **14.01.2026**
(21) Application number: 24382731.8
(22) Date of filing: 09.07.2024
(51) Int. Cl.: A23C 11/10, A23L 2/84, A23L 5/20, A23L 7/104, C12N 9/62

(54) **GLUTEN-FREE PLANT-BASED BEVERAGE**

(71) Applicant: Cygyc Biocon, S.L., 08520 Les Franqueses del Vallès (ES)
(72) Inventor: GUTIERREZ MONTERO, Alba, 08415 BIGUES I RIELLS (ES); BAGOT ALCARAZ, Meritxell, 08013 BARCELONA (ES)
(74) Representative: Sugrañes, S.L.P.

(57) **Abstract**

The invention relates to a process for removing gluten from plant-based beverages, which comprises the treatment of said plant-based beverage with a specific enzyme mixture. The invention also relates to such enzyme mixture and to its use for gluten removal. The invention also relates to the gluten-free plant-based beverage obtainable with this process.

## Description

### TECHNICAL FIELD

The present invention relates to an enzymatic process for preparing a gluten-free plant-based beverage.

### BACKGROUND ART

Plant-based beverages are typically manufactured by water extraction of different types of plant seeds or grains. Some common plant-based beverages are, for example, soy, oat, flax, almond or rice drinks, among others. These plant-based beverages are becoming increasingly popular as replacement for dairy milk, for multiple reasons. For example, they are preferred over cow's milk by consumers with food restrictions caused by allergies, lactose intolerance, or specific diets, and they are also preferred due to lifestyle factors, such by followers of vegetarianism or veganism, or by people concerned about the environment.

On the other hand, gluten is a protein found in certain cereal grains, namely, in wheat, rye and barley, which can trigger adverse reactions in some people, namely, those suffering from celiac diseases or from other non-celiac gluten intolerance or sensitivity.

It has been shown that, even if the grains used for preparing most common plant-based beverages do not, in principle, contain gluten, they can nevertheless contain gluten because they are frequently produced in the same place as wheat, barley and rye and may become contaminated with these other grains.

Therefore, there is a concern related to the safety of plant-based drinks, namely, for avoiding allergies and intolerances in gluten sensitive or intolerant persons, as it cannot be completely guaranteed that the plant raw material used in their production is gluten-free.

Therefore, some methods have been developed to remove the gluten found in those drinks.

For example, a known method is based on the use of enzymes for removing gluten from several food products, including plant derived beverages. Thus, for example, in the review article Scherf et al., Novel approaches for enzymatic gluten degradation to create high-quality gluten-free products, Food Res. Int. 2018, 110, 62-72, a good number of enzymes suitable for gluten degradation, for removing it from gluten containing food, are reviewed.

More particularly, the international patent application WO-A-2023/275354 discloses a process for the preparation of ready-to-drink products derived from cereal components, which comprises a fermentation step and/or an enzymatic hydrolysis, and preferably both. A particular embodiment of this invention (disclosed in Example 22) relates to the preparation of a gluten-free ready-to-drink non-fermented oat product using an aminopeptidase enzyme, namely, Flavourzyme^{®} from Novozymes. It is stated that this particular enzyme is useful in the preparation of gluten-free ready-to-drink products.

The international patent application WO-A-2023/099052 discloses a method for obtaining an oat-derived beverage, starting from oat grain. The process includes a step of treating the liquid oat product (previously prepared by treatment with a glycosidase) with an enzyme mixture comprising a at least a protease, a deamidase and a transglutaminase, optionally in combination with an amyloglycosidase, namely, wherein the protease is an acid carboxy-peptidase (EC 3.4.16.6), the deamidase is a protein-glutaminase (EC 3.5.1.44), the transglutaminase is EC 2.3.2.13, and the amyloglycosidase is EC 3.2.1.3. It is stated that the combination of these particular enzymes yields a gluten-free liquid food product which is stable, has good organoleptic properties and remains stable to further treatments or addition of further components.

Therefore, it would be desirable to develop new enzyme mixtures, which would be able to effectively reduce or remove the gluten content in plant-based beverages.

### SUMMARY OF THE INVENTION

The object of the present invention is a process for removing gluten from plant-based beverages using an enzyme mixture.

Another aspect of the invention is said enzyme mixture.

Another aspect of the invention the use of said enzyme mixture for gluten removal in plant-based beverages.

### DETAILED DESCRIPTION OF THE INVENTION

The object of the present invention is a process for removing gluten from plant-based beverages comprising treating the plant-based beverage with an enzyme mixture comprising the following enzymes: bromelain, protein-glutamine glutaminase, protein-glutamine gamma-glutamyltransferase and at least one additional enzyme selected from leucyl aminopeptidase, aspergillopepsin I and oryzin.

The authors of the present invention have developed a particular enzyme mixture, which is outstandingly effective for gluten removal in plant-based beverages.

Along the present description, as well as in the claims, singular expressions, generally preceded by the articles "a", "an" or "the", are intended to include the plural forms as well, unless the context clearly indicates otherwise.

The terms "about" or "approximately" referred to amounts, as used herein, are meant to include the exact amount and also a certain deviation around the stated amount, namely of ±5%.

The numerical ranges disclosed herein, generally expressed as "from ... to ..." or as "between ... and ...", are meant to include any number falling within the ranges and also the lower and upper limits.

### Plant-based beverages

Plant-based beverages can be defined as aqueous extracts obtained through breakdown of plant material, namely, from cereals, pseudo-cereals, legumes oilseeds or nuts. They are typically obtained by extraction with water followed by homogenization of the fluids, resulting in particle size distribution generally in range 5-20 microns which imitates cow's milk appearance and consistence (Sehti et al., J. Food Sci. Technol., 2016, 53(9), 3408-3423). They are also commonly referred to as "plant milk".

The terms "beverage" or "drink" are used interchangeably herein. Therefore, "plant-based" or "plant-derived" or "plant" beverage/drink are used as synonyms.

The plant-based beverage includes for example, the following: oat, rice, corn, soy, peanut, lupin, cowpea, almond, hazelnut, pistachio, walnut, sesame, flax, hemp, sunflower, quinoa, teff, barley, malt and amaranth drinks, among others.

In an embodiment, the plant-based beverage is selected from oat, rice, corn, soy, barley, malt and sesame drinks, and preferably selected from oat, rice, soy, barley and malt drinks.

In a preferred embodiment, the plant-based beverage is selected from oat drink, rice drink and soy drink.

In another preferred embodiment, the plant-based beverage is selected from oat drink, barley drink and malt drink.

In a more preferred embodiment, the plant-based beverage is an oat-based beverage.

In general, the process used to prepare the plant-based beverage involves several common steps, including, for example, milling the grains, beans, seeds or nuts of the plant starting material, water treatment, heating, filtering, homogenizing, and, frequently, also enzyme treatment for hydrolysing some of their major components, such as proteins and polysaccharides. There are many possible variants and alternatives of the process for preparing plant-based beverages, to optimize the properties of the drink end-product, and particularly adapted to each specific plant product, for example, as discussed in Sehti *et al.*, *op.cit.*

Any plant-based beverage, irrespective of the process used in its preparation, is suitable as starting material for the process according to the present invention.

### Enzyme mixture

The process according to the present invention is based on the use of a particular enzyme mixture, which is outstandingly effective for gluten removal.

In particular, the enzyme mixture comprises the following enzymes: bromelain, protein-glutamine glutaminase, protein-glutamine gamma-glutamyltransferase and at least one additional enzyme selected from leucyl aminopeptidase, aspergillopepsin I and oryzin.

In an embodiment, the enzyme mixture consists of the following enzymes: bromelain, protein-glutamine glutaminase, protein-glutamine gamma-glutamyltransferase and at least one additional enzyme selected from leucyl aminopeptidase, aspergillopepsin I and oryzin.

All the enzymes used in the enzyme mixture according to the present invention are readily available from commercial sources, for example, from the company CYGYC BIOCON S.L.

### Bromelain

Bromelain is one of the components of the enzyme mixture according to the present invention. Bromelain is an endopeptidase extracted from pineapple plant (*Ananas comosus*, of the Bromeliaceae plant family).

The term bromelain includes two proteases, known as fruit bromelain (EC 3.4.22.33), which is the major endopeptidase in the fruit, and stem bromelain (EC 3.4.22.32), which is the major endopeptidase present in extracts of plant stem.

The term bromelain, as used herein, means either fruit bromelain, or stem bromelain or a mixture of fruit bromelain and stem bromelain, in any proportion. Other synonyms for this enzyme include bromelain A, bromelain B, bromelase, bromelin, ananase, extranase or pinase, among others (https://www.brenda-enzymes.org/, https://enzyme.expasy.org/).

Fruit and stem bromelain are described, for example, in: Rawlings, Neil D. and Salvesen, Guy (eds.), Handbook of Proteolytic Enzymes, Volume 1, London: Academic Press, Third Edition, 2013, ISBN: 978-0-12-407744-7, Chapter 424, Stem Bromelain, 1871-1873 and Chapter 425, Fruit Bromelain, 1874-1875.

### Protein-glutamine glutaminase

Protein-glutamine glutaminase is another of the components of the enzyme mixture according to the present invention, whose EC number is EC 3.5.1.44.

Other synonyms of this enzyme are glutaminylpeptide glutaminase or peptidoglutaminase II (https://www.brenda-enzymes.org/, https://enzyme.expasy.org/). This enzyme hydrolyses the amide group of glutamine residues of proteins.

### Protein-glutamine gamma-glutamyltransferase

Protein-glutamine gamma-glutamyltransferase is another of the components of the enzyme mixture according to the present invention, whose EC number is EC 2.3.2.13.

Other synonyms of this enzyme are fibrinoligase, glutaminylpeptide gamma-glutamyltransferase, polyamine transglutaminase, TGase or transglutaminase, among others (https://www.brenda-enzymes.org/, https://enzyme.expasy.org/).

As is well-known, this enzyme introduces covalent cross-links in proteins by forming a bond between lysine and glutamine residues, in particular, between the gamma-carboxamide groups of peptide-bound glutamine residues and the 6-amino-groups of protein- and peptide-bound lysine residues, to give intra- and inter-molecular N(6)-(5-glutamyl)lysine cross-links.

### Additional enzymes

The enzyme mixture according to the present invention comprises at least one additional enzyme selected from the group consisting of leucyl aminopeptidase, aspergillopepsin I, and oryzin.

In an embodiment, the enzyme mixture contains at least two additional enzymes selected from the group consisting of leucyl aminopeptidase, aspergillopepsin I, and oryzin.

In an embodiment, the enzyme mixture contains the three additional enzymes leucyl aminopeptidase, aspergillopepsin I, and oryzin.

Leucyl aminopeptidase (EC 3.4.11.1) is an exopeptidase, which preferentially catalyses the hydrolysis of leucine residues at the N-terminus of peptides and proteins. Synonyms for this enzyme are, for example, cytosol aminopeptidase, leucine aminopeptidase and peptidase S, among others. It is described, for example, in Rawlings, op. cit, Chapters 329-330, Leucyl Aminopeptidase (Animal), Leucyl Aminopeptidase (Plant), 1465-1476.

Aspergillopepsin I (EC 3.4.23.18), as is well-known in the art, is a proteolytic enzyme found in a variety of *Aspergillus* species. Synonyms of this enzyme are aspergillopepsin A, aspergillopepsin F, aspergillopeptidase A, awamorin, proctase B, protease type XIII, proteinase B or trypsinogen kinase, among others. It is described, for example, in Rawlings, *op.cit.*, Chapter 27, 135-141.

Oryzin (E.C 3.4.21.63) is also produced by fungal sources, for example, from *Aspergillus oryzae.* Alternative names for this enzyme are aspergillopeptidase B or aspergillus alkaline proteinase, among others. It is described, for example, in Rawlings *et al.*, *op.cit.*, Chapter 713, 3237-3239.

### Enzyme treatment

The step of treating the plant-based beverage with the enzyme mixture typically means adding the enzymes in a suitable amount to the plant-based beverage, using suitable incubation time and temperature.

The enzyme mixture can either be prepared in advance and added simultaneously to the plant-based beverage substrate or, alternatively, the component enzymes of the mixture can be added sequentially to the plant drink.

The incubation time generally ranges from about 1 hour to about 5 hours, preferably from about 1 hour to about 4 hours, more preferably from about 1.5 hours to about 3 hours, and still more preferably is about 2 hours.

The incubation temperature generally ranges from about 40 °C to about 80 °C, preferably from about 50 °C to about 70 °C, and more preferably is about 60 °C.

The amount of each enzyme used is not critical and may range, for example, from about 0.01 % w/v to about 10 % w/v.

Bromelain can be used, for example, in a concentration of from about 0.01 % w/v to about 1 % w/v, preferably from about 0.05 % w/v to about 0.5 % w/v, and more preferably from about 0.10 % w/v to about 0.25 % w/v.

Protein-glutamine glutaminase can be used, for example, in a concentration of from about 0.1 % w/v to about 10 % w/v, preferably from about 0.2 % w/v to about 5 % w/v, more preferably from about 0.5 % w/v to about 1 % w/v.

Protein-glutamine gamma-glutamyltransferase can be used, for example, in a concentration of from about 0.01 % w/v to about 2 % w/v, preferably from about 0.05 % w/v to about 5 % w/v, more preferably from about 0.1 % w/v to about 0.5 % w/v.

Leucyl aminopeptidase can be used, for example, in a concentration of from about 0.05 % w/v to about 5 % w/v, preferably from about 0.1 % w/v to about 2.5 % w/v, more preferably from about 0.2 % w/v to about 1 % w/v.

Aspergillopepsin I can be used, for example, in a concentration of from about 0.05 % w/v to about 5 % w/v, preferably from about 0.1 % w/v to about 2.5 % w/v, more preferably from about 0.2 % w/v to about 1 % w/v.

Oryzin can be used, for example, in a concentration of from about 0.05 % w/v to about 5 % w/v, preferably from about 0.1 % w/v to about 2.5 % w/v, more preferably from about 0.2 % w/v to about 1 % w/v.

The above percentages, expressed in % w/v mean g of enzyme in 100 mL of plant-based drink.

After the enzymatic treatment, the enzymes are typically inactivated, for example, by heating. The inactivation may be performed at about 100-130 °C, during a period of from about 15 minutes to 1 hour, for example, or using a standard UHT (ultra-high-temperature) process.

### Gluten-free plant-derived beverage

The process according to the present invention is intended for the removal of gluten from plant-based beverages. As used herein the term "remove" means reducing the amount of gluten originally found in the treated plant-based beverage.

The term "gluten-free" as used herein referred to the plant-based beverage, after being submitted to the process of the present invention, means a beverage with a reduced amount of gluten, typically of less than 20 ppm, more preferably less than 15 ppm, or still more preferably less than 10 ppm.

In accordance with the term "gluten-free" as used herein, different regulatory organisms, such as Codex Alimentarius, European Commission or the U.S. Food and Drug Administration, define as gluten-free the products having less than 20 ppm of gluten.

In Example 1, a series of commercially available oat drinks were submitted to a process according to the present invention. As shown in the example, in all samples the amount of gluten was significantly reduced, and gluten-free drinks were obtained.

In Example 2 it is shown that it is the particular enzyme mixture as defined in the present invention that is responsible for the gluten-reducing effect. It is remarkable that that when just one of the enzymes of the enzyme mixture is missing or is substituted with another different one, the gluten reducing effect is significantly reduced, even if the amount of the other enzymes are increased.

Therefore, another aspect of the present invention is an enzyme mixture comprising bromelain, protein-glutamine glutaminase, protein-glutamine gamma-glutamyltransferase and at least one additional enzyme selected from leucyl aminopeptidase, aspergillopepsin I and oryzin. Preferably, the enzyme mixture consists of bromelain, protein-glutamine glutaminase, protein-glutamine gamma-glutamyltransferase and at least one additional enzyme selected from leucyl aminopeptidase, aspergillopepsin I and oryzin.

In an embodiment, the enzyme mixture, as above defined, contains at least two additional enzymes selected from the group consisting of leucyl aminopeptidase, aspergillopepsin I, and oryzin.

In an embodiment, the enzyme mixture, as above defined additionally contains the three enzymes leucyl aminopeptidase, aspergillopepsin I, and oryzin.

The relative amount of each enzyme in the enzyme mixture is not critical. For example, suitable weigh ratio between the component enzymes in the enzyme mixture includes a weight ratio bromelain:protein-glutamine glutaminase:protein-glutamine gamma-glutamyltransferase:leucyl aminopeptidase /aspergillopepsin I/oryzin in the following ranges: (3-4):(10-30):1:(1-3)/(1-3)/(1-3). It is understood that, either if the enzyme mixture contains one, two or three of the enzymes leucyl aminopeptidase, aspergillopepsin I and oryzin, each one of them is added typically in the stated weight ratio (i.e., 1-3 as hereinabove stated relative to the other enzymes in the mixture).

Another aspect of the invention is the use of the above defined enzyme mixture for gluten removal in plant-based beverages.

Additionally, another aspect of the present invention is a gluten-free plant-based beverage obtainable according to the process of the present invention

The present invention involves the following embodiments:
1.- A process for removing gluten from plant-based beverages comprising treating the plant-based beverage with an enzyme mixture comprising the following enzymes: bromelain, protein-glutamine glutaminase, protein-glutamine gamma-glutamyltransferase and at least one additional enzyme selected from leucyl aminopeptidase, aspergillopepsin I and oryzin.
2.- Process according to embodiment 1, characterized in that bromelain is selected from fruit bromelain, stem bromelain or mixtures thereof.
3.- Process according to embodiments 1 or 2, characterized in that the plant-based beverage is selected from the group consisting of oat drink, rice drink, corn drink, soy drink, peanut drink, lupin drink, cowpea drink, almond drink, hazelnut drink, pistachio drink, walnut drink, sesame drink, flax drink, hemp drink, sunflower drink, quinoa drink, teff drink, barley drink, malt drink and amaranth drink.
4.- Process according to embodiment 3, characterized in that the plant-based beverage is selected from the group consisting of oat drink, rice drink, corn drink, soy drink, barley drink, malt drink and sesame drink, preferably selected from the group consisting of oat drink, barley drink, malt drink, rice drink and soy drink.
5. Process according to embodiment 4, characterized in that the plant-based beverage is selected from the group consisting of oat drink, barley drink, and malt drink.
6. Process according to embodiment 4, characterized in that the plant-based beverage is selected from the group consisting of oat drink, rice drink and soy drink.
7.- Process according to any one of embodiments 4 to 6, characterized in that the plant-based beverage is an oat-based beverage.
8.- Process according to any one of embodiments 1 to 7, characterized in that the enzyme mixture consists of the following enzymes: bromelain, protein-glutamine glutaminase, protein-glutamine gamma-glutamyltransferase and at least one additional enzyme selected from leucyl aminopeptidase, aspergillopepsin I and oryzin.
9.- Process according to any one of embodiments 1 to 8, characterized in that the enzyme mixture contains at least two enzymes selected from the group consisting of leucyl aminopeptidase, aspergillopepsin I, and oryzin.
10.- Process according to any one of embodiments 1 to 9, characterized in that the enzyme mixture contains the three enzymes leucyl aminopeptidase, aspergillopepsin I, and oryzin.
11.- Process according to any one of embodiments 1 to 10, characterized in that the plant-based beverage is incubated with the enzyme mixture during a period of time comprised between 1 hour and 5 hours, at a temperature comprised between 40 °C and 80 °C.
12.- Process according to embodiment 11, characterized in that the incubation time is comprised between 1 hour and 4 hours, preferably comprised between 1.5 hours and 3 hours, and more preferably is about 2 hours.
13.- Process according to embodiments 11 or 12, characterized in that the incubation temperature is comprised between 50 °C and 70 °C, and preferably is about 60 °C.
14.- Process according to anyone of embodiments 1 to 13, characterized in that each enzyme in the enzyme mixture is used in a concentration comprised between 0.01 % w/v and 10 % w/v.
15.- Process according to embodiment 14, characterized in that bromelain is used in a concentration comprised between 0.01 % w/v and 1 % w/v, preferably between 0.05 % w/v and 0.5 % w/v, and more preferably between 0.10 % w/v and 0.25 % w/v.
16.- Process according to embodiments 14 or 15, characterized in that protein-glutamine glutaminase is used in a concentration comprised between 0.1 % w/v and 10 % w/v, preferably between 0.2 % w/v and 5 % w/v, and more preferably between 0.5 % w/v and 1 % w/v.
17.- Process according to any one of embodiments 14 to 16, characterized in that protein-glutamine gamma-glutamyltransferase is used in a concentration comprised between 0.01 % w/v and 2 % w/v, preferably between 0.05 % w/v and 5 % w/v, and more preferably between 0.1 % w/v and 0.5 % w/v.
18.- Process according to any one of embodiments 14 to 17, characterized in that leucyl aminopeptidase is used in a concentration comprised between 0.05 % w/v and 5 % w/v, preferably between 0.1 % w/v and 2.5 % w/v, and more preferably between 0.2 % w/v and 1 % w/v.
19.- Process according to any one of embodiments 14 to 18, characterized in that aspergillopepsin I is used in a concentration comprised between 0.05 % w/v and 5 % w/v, preferably between 0.1 % w/v and 2.5 % w/v, and more preferably between 0.2 % w/v and 1 % w/v.
20.- Process according to any one of embodiments 14 to 19, characterized in that oryzin is used in a concentration comprised between 0.05 % w/v and 5 % w/v, preferably between 0.1 % w/v and 2.5 % w/v, and more preferably between 0.2 % w/v and 1 % w/v.
21.- Enzyme mixture comprising bromelain, protein-glutamine glutaminase, protein-glutamine gamma-glutamyltransferase and at least one additional enzyme selected from leucyl aminopeptidase, aspergillopepsin I and oryzin.
22.- Enzyme mixture according to embodiment 21, characterized in that bromelain is selected from fruit bromelain, stem bromelain and mixtures thereof.
23.- Enzyme mixture according to embodiments 21 or 22, characterized in that it consists of bromelain, protein-glutamine glutaminase, protein-glutamine gamma-glutamyltransferase and at least one additional enzyme selected from leucyl aminopeptidase, aspergillopepsin I and oryzin.
24.- Enzyme mixture according to embodiments 21 to 23, characterized in that it contains at least two additional enzymes selected from leucyl aminopeptidase, aspergillopepsin I and oryzin.
25.- Enzyme mixture according to embodiments 21 to 24, characterized in that it contains the three enzymes leucyl aminopeptidase, aspergillopepsin I and oryzin.
26.- Enzyme mixture according to embodiments 21 to 25, characterized in that the weight ratio bromelain:protein-glutamine glutaminase:protein-glutamine gamma-glutamyltransferase:leucyl aminopeptidase /aspergillopepsin I/oryzin in the enzyme mixture is comprised in the range (3-4):(10-30):1:(1-3)/(1-3)/(1-3).
27.- Use of the enzyme mixture according to embodiments 21 to 26 for gluten removal in plant-based beverages.

### Examples

### Example 1. - Use of the process according to the present invention in commercially available oat drinks

Different commercially available oat drinks, named as Samples 1 to 7 in Table 1, were first analysed to determine their gluten concentration, and then subjected to the process according to the present invention. After being treated, the oat-derived drinks were again analysed to determine the gluten content.

**TABLE 1**

| Commercial samples | Gluten (ppm) before treatment | Gluten (ppm) after treatment |
|---|---|---|
| Sample-1 | 40 | 18 |
| Sample-2 | 23 | <10 |
| Sample-3 | 20 | <10 |
| Sample-4 | 79 | <10 |
| Sample-5 | 23 | <10 |
| Sample-6 | 22 | <10 |
| Sample-7 | 29 | <10 |

The method of the invention was performed as follows: each oat milk sample was incubated with the enzyme mixture at 60 °C for 2 h. The following enzymes were sequentially added, at the following concentrations:
- Fruit bromelain: 14 mg/mL
- Protein-glutamine glutaminase: 67 mg/mL
- Protein-glutamine gamma-glutamyltransferase: 3 mg/mL
- Leucyl aminopeptidase: 5 mg/mL
- Aspergillopepsin I: 5 mg/mL
- Oryzin: 5 mg/mL

After the enzymatic treatment, enzymes were inactivated by heating at 100 °C for 10 minutes.

The gluten content was determined by competitive ELISA assay, using a kit from R-Biopharm (RIDASCREEN^{®} Gliadin competitive R7021), following manufacturer's instructions. It is a competitive enzyme immunoassay for the quantitative determination of prolamin peptide fragments from wheat (gliadin), rye (secalin) and barley (hordein) in foods. It is a validated AOAC official method of analysis (AOAC-OMA) and is based on the use of the R5 monoclonal antibody (Lacorn et al., J AOAC Int. 2015, 98 (5), 1346-1354).

### Example 2. - Comparative efficacy of different enzyme mixtures for gluten removal

For this assay a commercially available oat drink (Sample 1 used in the Example 1) was used. In this case, the drink was enriched with additional gluten, to reach a gluten content of 120 ppm (gluten used was "Gluten from wheat", Sigma Aldrich).

In Tables 2 and 3 the results of the comparative assays are shown, wherein the column "INV" corresponds to the enzyme mixture according to the present invention and columns C1 to C11 correspond to comparative mixtures 1 to 11.

**TABLE 2**

| | *Samples* / *enzyme concentration (g*/*mL)* | | | | | |
|---|---|---|---|---|---|---|
| *Enzymes* | **INV** | C1 | C2 | C3 | C4 | C5 |
| Bromelain | **14** | - | 14 | - | - | 28 |
| Protein-glutamine glutaminase | **67** | 67 | - | 67 | - | - |
| Protein-glutamine gamma-glutamyltransferase | **3** | 143 | 140 | 3 | 280 | - |
| Leucyl aminopeptidase | **5** | 5 | 9 | 5 | 9 | 5 |
| Aspergillopepsin I | **5** | 5 | 9 | 5 | 9 | 5 |
| Oryzin | **5** | 5 | 9 | 5 | 9 | 5 |

| | *Gluten content (ppm)* | | | | | |
|---|---|---|---|---|---|---|
| *Before treatment* | **120** | 120 | 120 | 120 | 120 | 120 |
| *After treatment* | **18** | 54 | 46 | 57 | 44 | 72 |

**TABLE 3**

| | *Samples* / *enzyme concentration (g*/*mL)* | | | | | | |
|---|---|---|---|---|---|---|---|
| *Enzymes* | **INV** | C6 | C7 | C8 | C9 | C10 | C11 |
| Bromelain | **14** | 14 | 14 | 28 | - | 14 | 14 |
| Protein-glutamine glutaminase | **67** | - | 67 | - | 134 | - | - |
| Protein-glutamine gamma-glutamyltransferase | **3** | 280 | 3 | - | 6 | - | - |
| Leucyl aminopeptidase | **5** | - | - | - | - | 5 | - |
| Aspergillopepsin I | **5** | - | - | - | - | 5 | - |
| Oryzin | **5** | - | - | - | - | 5 | - |
| Glutaminase (EC 3.5.1.2) | | | | | | 35 | 35 |

| | *Gluten content (ppm)* | | | | | | |
|---|---|---|---|---|---|---|---|
| *Before treatment* | **120** | 120 | 120 | 120 | 120 | 120 | 120 |
| *After treatment* | **18** | 67 | 62 | 97 | 56 | 76 | 107 |

As can be observed, for comparison purposes, some of the enzymes of the enzyme mixture according to the invention are omitted (optionally then increasing the amount of the other enzymes). In comparatives C10 and C11 an alternative enzyme (glutaminase EC 3.5.1.2) is used, instead of protein-glutamine glutaminase and protein-glutamine gamma-glutamyltransferase.

The incubation with the enzyme mixtures was performed in the same conditions as disclosed in Example 1 (but using the enzymes and concentrations shown in Tables 2 and 3). After incubation, the enzymes were inactivated in the same way.

## Claims

1. A process for removing gluten from plant-based beverages comprising treating the plant-based beverage with an enzyme mixture comprising the following enzymes: bromelain, protein-glutamine glutaminase, protein-glutamine gamma-glutamyltransferase and at least one additional enzyme selected from leucyl aminopeptidase, aspergillopepsin I and oryzin.

2. Process according to claim 1, **characterized in that** the plant-based beverage is selected from the group consisting of oat drink, rice drink, corn drink, soy drink, peanut drink, lupin drink, cowpea drink, almond drink, hazelnut drink, pistachio drink, walnut drink, sesame drink, flax drink, hemp drink, sunflower drink, quinoa drink, teff drink, barley drink, malt drink and amaranth drink.

3. Process according to claim 2, **characterized in that** the plant-based beverage is selected from the group consisting of oat drink, rice drink, corn drink, soy drink, barley drink, malt drink and sesame drink, and preferably is an oat drink.

4. Process according to any one of claims 1 to 3, **characterized in that** the enzyme mixture consists of the following enzymes: bromelain, protein-glutamine glutaminase, protein-glutamine gamma-glutamyltransferase and at least one additional enzyme selected from leucyl aminopeptidase, aspergillopepsin I and oryzin.

5. Process according to any one of claims 1 to 4, **characterized in that** the enzyme mixture contains at least two enzymes selected from the group consisting of leucyl aminopeptidase, aspergillopepsin I, and oryzin.

6. Process according to any one of claims 1 to 5, **characterized in that** the enzyme mixture contains the three enzymes leucyl aminopeptidase, aspergillopepsin I, and oryzin.

7. Process according to any one of claims 1 to 6, **characterized in that** the plant-based beverage is incubated with the enzyme mixture during a period of time comprised between 1 hour and 5 hours, at a temperature comprised between 40 °C and 80 °C.

8. Process according to anyone of claims 1 to 7, **characterized in that** each enzyme in the enzyme mixture is used in a concentration comprised between 0.01 % w/v and 10 % w/v.

9. Process according to claim 8, **characterized in that** bromelain is used in a concentration comprised between 0.01 % w/v and 1 % w/v, preferably between 0.05 % w/v and 0.5 % w/v, and more preferably between 0.10 % w/v and 0.25 % w/v.

10. Process according to claims 8 or 9, **characterized in that** protein-glutamine glutaminase is used in a concentration comprised between 0.1 % w/v and 10 % w/v, preferably between 0.2 % w/v and 5 % w/v, and more preferably between 0.5 % w/v and 1 % w/v.

11. Process according to any one of claims 8 to 10, **characterized in that** protein-glutamine gamma-glutamyltransferase is used in a concentration comprised between 0.01 % w/v and 2 % w/v, preferably between 0.05 % w/v and 5 % w/v, and more preferably between 0.1 % w/v and 0.5 % w/v.

12. Enzyme mixture comprising bromelain, protein-glutamine glutaminase, protein-glutamine gamma-glutamyltransferase and at least one additional enzyme selected from leucyl aminopeptidase, aspergillopepsin I and oryzin.

13. Enzyme mixture according to claim 12, **characterized in that** it consists of bromelain, protein-glutamine glutaminase, protein-glutamine gamma-glutamyltransferase and at least one additional enzyme selected from leucyl aminopeptidase, aspergillopepsin I and oryzin.

14. Enzyme mixture according to claims 12 or 13, **characterized in that** the weight ratio bromelain:protein-glutamine glutaminase:protein-glutamine gamma-glutamyltransferase:leucyl aminopeptidase /aspergillopepsin I/oryzin in the enzyme mixture is comprised in the range (3-4):(10-30):1:(1-3)/(1-3)/(1-3).

15. Use of the enzyme mixture according to claims 12 to 14 for gluten removal in plant-based beverages.
